Europäisches Patentamt

European Patent Office

Office européen des brevets

(11) Publication number: **0 484 680 A1**

# EUROPEAN PATENT APPLICATION

(21) Application number: **91117058.7**

(22) Date of filing: **07.10.91**

(51) Int. Cl.5: **A61M  25/00**

(30) Priority: **09.10.90 JP 271487/90**
    **09.10.90 JP 271488/90**
    **31.01.91 JP 31724/91**

(43) Date of publication of application:
    **13.05.92 Bulletin  92/20**

(84) Designated Contracting States:
    **DE FR GB**

(71) Applicant: **THE FURUKAWA ELECTRIC CO.,
    LTD.
    6-1, Marunouchi 2-chome Chiyoda-ku
    Tokyo 100(JP)**

(72) Inventor: **Ogiwara, Yoshiaki
    610-4-52, Kiyotaki-Tanze-machi
    Nikko-shi, Tochigi-ken(JP)**
    Inventor: **Yasuhara, Masaki
    89-25, Kujira-machi
    Nikko-shi, Tochigi-ken(JP)**
    Inventor: **Shirakawa, Ryotomo
    338-8, Senbongi
    Imaichi-shi, Tochigi-ken(JP)**
    Inventor: **Yura, Seiya
    2-16-2, Tsurumaki
    Setagaya-ku, Tokyo(JP)**

(74) Representative: **Bühling, Gerhard, Dipl.-Chem.
    et al
    Patentanwaltsbüro Tiedtke-Bühling-Kinne
    Grupe-Pellmann-Grams Bavariaring 4
    W-8000 München 2(DE)**

(54) Method of producing catheter and catheter thus obtained.

(57) A method of producing catheter is provided, comprising the steps of molding a catheter with rubber or plastic, subjecting at least a portion of said catheter touching on human body to activation treatment, and then forming a metallic layer having bactericidal or sterile action on the main body of catheter by electroless plating treatment. Other methods and a catheter thus obtained are also claimed.

EP 0 484 680 A1

Fig. 1

## BACKGROUND OF THE INVENTION

The present invention relates to a method of producing catheter with a metallic layer having bactericidal or sterile action formed on the wall of the main body of medical catheter usable for ejecting fluid accumulated in the body cavity or inversely injecting fluid into the body cavity, particularly on the wall of the main body of ureteral catheter to be inserted into bladder via urethra, and a catheter obtainable in such a way.

Cerebrospinal diseases, for example, cerebral hemorrhage, encephalomalacia, spinal damage, etc. are often accompanied with the symptoms such as dysuria and incontinence of urine. Hence, the ureteral catheter is inserted into the bladder to facilitate the micturition by leaving it there. Moreover, for the patients after operation, various catheters are used for ejecting fluid, pus, etc. accumulated in the body cavity as well as aid of the micturition and these catheters are most frequently used by leaving them in the body cavity because of the necessity.

As shown in Fig. 1 and Fig. 2, this catheter has a long single structure formed with raw rubber, wherein a central cave (4) is formed through the center of a shaft (1) in the axial direction to introduce the urine entering from a side hole (2) at the tip to a discharge port (3) disposed outside the body, and further a balloon cave (6) is provided in the shaft (1) in the axial direction to form a balloon (5) by expanding the tip portion of shaft (1)for allowing said side hole (2) portion to stay in the bladder (8) as Shown in Fig. 3. Into this balloon cave (6), water,etc. are injected from an injection port (7) with valve structure provided on the side of discharge port (3) to spherically expand the balloon (5), thereby engaging said balloon (5) in the bladder (8).

Here, a problem in holding the catheter in the body cavity as mentioned is an infectious disease due to bacteria.

Particularly with the ureteral catheter, because it stays in the urethra for relatively long hours, the bacteria adhering to the anus, genitalia, etc. often invade into the bladder through catheter to make a cause inducing the cystitis. In particular, in the case of female, because the urethra is thicker, shorter and more straight over male, this infection by bacteria occurs very frequently.

In order to prevent this invasion of bacteria into bladder, a proposal has been made conventionally to attach a metal ring or coil with bactericidal action or sterile action to the tube of catheter (refer to Japanese Patent Publication No. Sho 54-14876).

By said method of attaching metal ring or coil, however, the tube portion of catheter becomes thick because of thick metal ring etc. resulting in an more pain in the patient on insertion and, in addition, fixing of metal ring etc. onto a fixed position of the tube portion of catheter was very difficult.

Moreover, the main body of catheter is molded hitherto in a shape of tube using rubbers such as raw rubber etc. as main raw materials and then coated with resins such as Teflon etc. to relieve the stimulus to human body, but this coating process has increased the production cost.

The purpose of the invention is to solve the problems of conventional catheter as mentioned above and to provide a catheter which allows a patient to feel no pain and which retains the effect of prevention from infection for a long term at very low cost.

## SUMMARY OF THE INVENTION

For achieving this purpose, the production method of the catheter of the invention is characterized by molding a catheter with rubber or plastic, subjecting at least a portion of said catheter to be thrusted in human body to activation treatment after surface treatment on alkalin aqueous solution of alcohol or without said treatment, and then forming a metallic layer having bactericidal or sterile action on the main body of catheter by electroless plating treatment, and, in this case, further heat treatment of the catheter formed with said metallic layer at 30 to 150 ° C results in better effectiveness.

Moreover, another production method of the invention is characterized by molding a catheter with rubber or plastic, subjecting the surface layer of at least a portion of said catheter touching with human body to curing reaction to a state of half-cure, forming a metallic layer having bactericidal or sterile action on said half-cured surface layer by electroless plating treatment or forming a silver film on said half-cured surface layer by treatment of silver mirror reaction, and then curing said half-cured portion to a desired hardness.

Furthermore, in either case above, the surface of molded catheter may be preferably coated with Teflon beforehand.

Still more, the catheter of the invention is one obtained by the methods as above.

BRIEF DESCRIPTION OF THE DRAWING

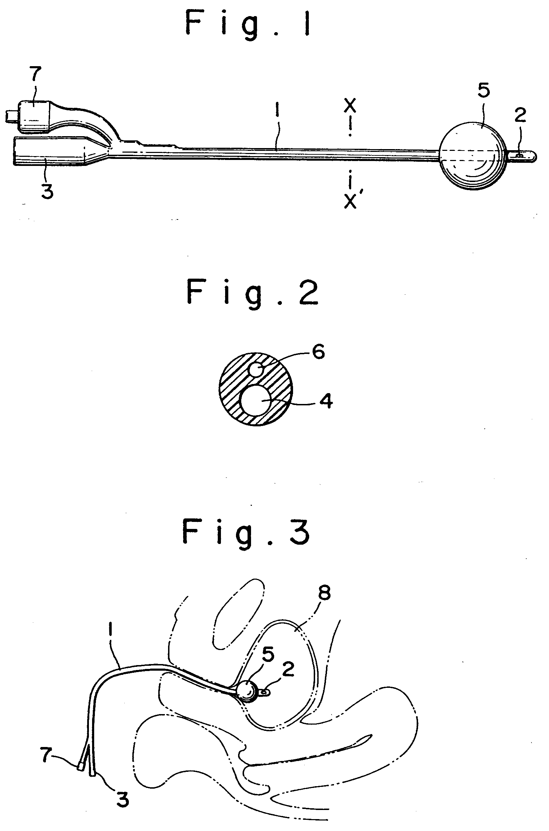

Fig. 1 is a side view of catheter, Fig. 2 is a cross section on X-X' line of Fig. 1, and Fig. 3 is an illustration diagram of the state of catheter inserted into human body.

DETAILED DESCRIPTION OF THE INVENTION

According to the invention, metallic thin films of, for example, copper, gold and silver having bactericidal or sterile action can be provided on the surface of at least a portion of catheter tube touching with human body (i.e. portion to be inserted into human body) by electroless plating treatment, hence very thin layer of metal can be adhered, so that the tube portion of catheter does not become thick (even if adhered onto the inner wall of tube, the inner diameter of tube should not be narrowed down).

Further, in this case, heat treatment immediately after electroless plating treatment is more effective.

This is because released metallic ions can be available from the metallic layer coalesced into the main body of a catheter over a long term, with very little possibility of the metal-separation due to the heat treatment immediately after electroless plating.

Moreover, in another one of the invention, the main body of catheter is formed with rubber or plastic, at least a portion of forming metallic film is given with electroless plating treatment in a state of half-cure or this portion is given with treatment of silver mirror reaction in the case of particularly forming silver film, and then said metal film-adhered portion is cured, hence the metal to be plated coalesces with resin, so that the release of metallic ions becomes also possible over a long term without easily dropping out.

The catheter of the invention therefore has almost the same thickness as the commercial catheter produced with rubber or plastic and further thin metallic layer that is incomparable to catheter attached with said metal ring,etc. is provided. Consequently, it inflicts no pain on patient and yet causes no infectious diseases coincidentaly, even if it may stay in the body cavity over a long term, serving also as the prevention from infectious diseases.

Besides, in the invention, since the metallic film which does not give a stimulus to the human body is provided on at least a portion of the main body of catheter to be inserted into the body cavity by electroless plating treatment, it becomes possible to use rubber catheter that has been unavailable in bare state because of the stimulus to human body in the naked state, hence it can take place of Teflon, coat on the surface of rubber as in the past, thereby producing an advantage to provide the rubber catheter at low cost.

In following, the invention will be illustrated in detail.

First, in one of the inventions, after the main body of catheter comprising rubber or plastic is masked leaving a portion needed for plating treatment, Pd, which becomes a catalyst on electroless plating treatment, is adsorbed by activation treatment.

When the main body of catheter adsorbed Pd is dipped into the electroless plating solution, metals deposit with Pd serving as a nucleus to form metals with bactericidal action or sterile action, for example, Cu, Ni, Ag, etc. The metal species to be formed by electroless plating treatment are not particularly confined, so they can be any mental with bactericidal action or sterile action such as Au etc. other than above-mentioned. Besides, Au can easily be plated by replacing with Au after formed the layer of Cu etc.

The thickness of the metallic layer formed by electroless plating treatment in such away is around 0.2 to 0.6 $\mu$m.

At this time, depending on the kind of rubbers, electroless plating treatment cannot always be made well by direct activation treatment. In such a case, the surface is treated beforehand with alkaline aqueous solution of alcohol as follows: For instance, the wall of the main body of catheter is allowed to swell by dipping it into a surface treatment liquor for catheter containing not less than in and not more than ion alkali (for example, NaOH and KOH) and not less than 5 vol. % and not more than 50 vol. % alcohol, the balance being water, to extend the spaces between molecules of resin constituting the catheter and simultaneously to introduce the hydrophilic groups.

Here, it is because the swelling force of resin could be too weak, if either alkali-component or alcohol is lacking in volume, and also, the swelling force of resin could be so strong that the overall resin degrades, if both components go over the limits of volume, that the concentrations of Alkali and Alcohol are set bounded.

Besides, when the adhesive force of the metallic layer formed by electroless plating treatment is weak, metal comes to penetrate into the surface of resin, thus preventing the dropping-out by heat treatment for 10 to 180 minutes at 30 to 150 °C. The temperature and the time required for the heat treatment vary depending on the kind of resins constituting the main body of catheter and the kind of metals formed on the surface of catheter, hence it is necessary to experimentally determine them through the combinations thereof.

Moreover, another invention is a production method of catheter, wherein the main body of catheter molded with rubber or plastic is made into a state of half-cure by heat treatment, such main body of catheter in half-cured state is masked, if necessary, leaving a portion needed for the electroless plating treatment or particularly for the treatment of silver mirror reaction, metal is allowed to deposit by electroless plating treatment or treatment of silver mirror reaction, and then overall portions are further treated under heat to cure to a desired hardness.

In this way, by giving the electroless plating or treatment of silver mirror reaction to the main body of catheter in half-curing state and then by completely curing it, the metallic film deposited on the surface of catheter comes to firmly adhere to resin, leading to hard peeling -off of metallic film on the use or during the use of catheter.

Depending on the resins to mold the main body of catheter, the adhesion of metal is sometimes poor by direct electroless plating treatment. In such a case, the molded main body of catheter in half-curing state may preferably be treated with alkaline aqueous solution of alcohol as mentioned above.

Besides, since a side hole communicating to the internal portion of tube is provided at the tip of the main body of catheter, if giving the electroless plating without masking this hole, it goes without saying that the metallic layer can be formed also on the inner wall of the main body of catheter.

Example 1

The main body of catheter comprising raw rubber was activated with activator 444 (made by MELTEX Co.) with following composition to adsorb active Pd onto the surface. Then, this was given with electroless Cu plating by dipping into Noviganth HC (made by Nippon Schering Co.) with following composition to form about 0.2 $\mu$m thick copper layer on the surface of the main body of catheter. While the copper layer was adhered onto the surface of catheter without peeling-off even in this state, the main body of catheter was kept in air bath for 50 minutes at 80 °C to further increase the adhesive strength, thus penetrating the copper layer into rubber and producing a catheter having copper layer on the surface.

The copper adhered onto the surface of rubber was in uniform film-like state (microscopically, copper powder was uniformly adhered) and, even when inserted into human body, the catheter neither gave a stimulus to the human body nor damaged the inside of body cavity.

| • Nobigant HC | |
| --- | --- |
| Copper sulfate pentahydrate | 15 g/L |
| EDTA-4Na | 28 g/L |
| Formalin | 10 mL/L |
| Sodium hydroxide | 4 g/L |
| Additive | |

| • Activator 444 | |
| --- | --- |
| Palladium potassium chloride | 0.5 g/L |
| Tin chloride | 25 g/L |
| Hydrochloric acid | 300 mL/L |

Example 2

The main body of catheter comprising raw rubber was activated with said activator 444 to adsorb active Pd onto surface. Then, this was given with electroless Ni plating by dipping into BEL-801 (made by Uemura Kogyo Co.) with following composition to form about 0.6 $\mu$m thick nickel layer on the surface of the main body of catheter. While the nickel layer was adhered onto the surface of catheter without peeling-off even in this state, the main body of catheter was kept in air bath for 60 minutes at 80 °C to further increase the adhesive strength, thus penetrating the nickel layer into rubber and producing a catheter having nickel layer on the surface. Through these treatments, the nickel layer was uniformly formed on the surface of the main body of catheter. Even when inserted this catheter into human body, it neither gave a stimulus to the human body nor damaged the inside of body cavity.

| • BEL-801 | |
|---|---|
| Nickel sulfact hexahydrate | 30 g/L |
| Sodium dihydrogen citrate | 10 g/L |
| Sodium succinate | 20 g/L |
| Sodium acetate | 20 g/L |
| DMAB | 5 mL/L |
| Additive | |

Example 3

After the Foley catheter (made by Nippon Sherwood Co.) comprising raw rubber was dipped into a surface treatment liquor for catheter with composition shown in Table 1 for 30 seconds at 30 °C to swell the surface of rubber, it was activated with said activator 444 to adsorb active Pd onto the surface. Then, this was given with electroless Cu plating by dipping into said Noviganth HC to form about 0.2 $\mu$m thick copper layer on the surface of the main body of catheter. while the copper layer was adhered onto the surface of catheter without peeling-off even in this state, the main body of catheter was kept in air bath for 50 minutes at 80 °C to further increase the adhesive strength, thus penetrating the copper layer into resin and producing a catheter having copper layer on the surface.

Of these catheters, the surface state and the degree of adhesion of copper layer were investigated, the results of which are shown in Table 1.

Table 1

| | Solution No. | Concentration of NaOH (N) | Concentration of ethanol (vol. %) | Results (Surface state and degree of adhesion) |
|---|---|---|---|---|
| Inventive example | 1 | 1. 20 | 7. 00 | Good |
| | 2 | 1. 25 | 48. 00 | Good |
| | 3 | 9. 85 | 6. 50 | Good |
| | 4 | 9. 85 | 48. 50 | Good |
| | 5 | 5. 00 | 25. 00 | Good |
| Comparative example | 6 | 0. 50 | 48. 00 | No good |
| | 7 | 0. 50 | 6. 50 | No adherence |
| | 8 | 11. 00 | 48. 00 | No good |
| | 9 | 5. 00 | 55. 00 | No good |

Example 4

After said Foley catheter comprising raw rubber was dipped into a surface treatment liquor for catheter with composition of the invention shown in Table 1 for 30 seconds at 30 ° C to swell the surface of rubber, it was activated with said activator 444 to adsorb active Pd onto the surface. Then, this was given with electroless Ni plating by dipping into said BEL-801 (Uemura Kogyo Co.) to form about 0.6 $\mu$m thick nickel layer on the surface of the main body of catheter. While the nickel layer was adhered onto the surface of catheter without peeling-off even in this state, the main body of catheter was kept in air bath for 60 minutes at 80 ° C to further increase the adhesive strength, thus penetrating the nickel layer into resin and producing a catheter having nickel layer on the surface. Through these treatments, the nickel layer was uniformly formed on the surface of the main body of catheter. Even when inserted this catheter into the human body, it neither gave a stimulus to the human body nor damaged the inside of body cavity.

Example 5

After said Foley catheter was dipped into a surface treatment liquor for catheter with composition concerned with the invention shown in Table 1 for 30 seconds at 30 ° C to swell the surface of resin, it was activated with said activator 444 to adsorb active Pd onto the surface. Then, this was given with electroless Cu plating by dipping into said Noviganth HC to perform about 0.2 $\mu$m thick copper plating on the surface of the main body of catheter. Further, the main body of catheter was dipped into GOLD-8 (made by World Metal Co.) with following composition to replace copper with gold and it was kept in air bath for 60 minutes at 80 ° C to increase the adhesive strength, thus uniformly adhering the layer of gold.

| • GOLD-8 | |
|---|---|
| Potassium gold cyanide | 0.5 g/L |
| Sodium cyanide | 4 g/L |
| Sodium carbonate | 5 g/L |
| Additive | |

Example 6

After about 0.2 μm thick copper plating was given onto the surface of the main body of catheter by electroless Cu plating in Example 1, the main body of catheter was dipped into said GOLD-8 to replace copper with gold and it was kept in air bath for 60 minutes at 80 ° C to increase the adhesive strengh, thus uniformly adhering the layer of gold. Even when inserted this catheter into the human body, it neither gave a stimulus to the human body nor damaged the inside of body cavity.

Example 7

After molded the main body of catheter with raw rubber, this was halfcured to about 60 % by heat treatment. This main body of catheter in half-curing state was activated with said activator 444 to adsorb active Pd onto the surface. Then, it was given with electroless Cu plating by dipping into said Noviganth HC to form about 0.2 μm thick copper film on the surface of the main body of catheter. following this, heat treatment was made again to cure to a desired hardness. With the catheter produced in this way, uniform copper film was firmly adhered (microscopically, copper powder was adhered uniformly) onto the surface of rubber.

Example 8

After molded the main body of catheter with raw rubber, this was half-cured to about 75 % by heat treatment. This main body of catheter in half-curing state was dipped into a surface treatment solution for catheter comprising 2N NaOH + 25 vol. % $C_2H_5OH$ to introduce the hydrophilic groups onto the surface of the main body of catheter, and then activated with said activator 444 to adsorb active Pd onto the surface. Then, it was given with electroless Cu plating by dipping into said Noviganth HC to form about 0.4 μm thick copper film on the surface of the main body of catheter. Following this, heat treatment was made again to cure to a desired hardness. With the catheter thus treated, the copper film can be attached thickly and yet the flexibility inherent in catheter is also not hindered.

Example 9

The main body of catheter molded with silicone resin was cured to about 70 % by heat treatment. This half-cured main body of catheter was masked except a portion of tube to be inserted into the human body and activated with activator 444 to adsorb active Pd on the surface. Then, this was given with electroless Cu plating by dipping into said Noviganth HC to form about 0.3 μm thick copper film on the surface of the main body of catheter. Following this, heat treatment was made again to cure to a desired hardness. With the catheter produced in such way, uniform copper film was firmly adhered (microscopically, copper powder was adhered uniformly) onto the surface of rubber.

Example 10

After molded the main body of catheter with raw rubber, it was half-cured to about 60 % by heat treatment. This main body of catheter in half-curing state was activated with said activator 444 to adsorb active Pd on the surface. Then, this was given with electroless Ni plating by dipping into said BEL-801 to form about 0.2 μm thick nickel film on the surface of the main body of catheter. Following this, heat treatment was made again to cure to a desired hardness. With the catheter produced in such way, uniform nickel film was firmly adhered (microscopically, nickel powder was adhered uniformly) onto the surface of rubber.

Example 11

After molded the main body of catheter with raw rubber, it was half-cured to about 60 % by heat treatment. this main body of catheter in half-curing state was dipped into a treatment solution for silver mirror reaction in which an appropriate amount of aqueous ammonia was added to 20 g of silver nitrate, 100 g of sodium potassium tartrate was added to this and the total volume was diluted with water so as to become 1700 mL to form about 0.2 $\mu$m thick silver film on the surface of the main body of catheter. Following this, heat treatment was made again to cure to a desired hardness. With the catheter produced in such way, uniform silver film was firmly adhered (microscopically, silver powder was adhered uniformly) onto the surface of rubber.

Example 12

After molded the main body of catheter with raw rubber, it was half-cured to about 75 % by heat treatment. This main body of catheter in half-curing state was dipped into a surface treatment solution for catheter comprising 2N NaOH + 25 vol. % $C_2H_5OH$ to introduce hydrophilic groups onto the surface of the main body of catheter, and thereafter about 0.4 $\mu$m thick silver was deposited onto the surface of the main body of catheter by Brashear method (3.5 g silver nitrate + appropriate amount of aqueous ammonia + 2.5 g sodium hydroxide + 45 g grape sugar + 4 g tartaric acid + 100 mL alcohol + 1000 mL water). Following this, heat treatment was made again to cure to a desired hardness. With the catheter thus treated, the silver film can be attached thickly and yet the flexibility inherent in catheter is also not hindered.

Example 13

The main body of catheter molded with silicone resin was cured to about 70 % by heat treatment. This half-cured main body of catheter was masked except a portion of tube to be inserted into the human body and dipped into a treatment solution for silver mirror reaction comprising 3.5 g silver nitrate + appropriate amount of aqueous ammonia + 1.2 mL 38% formaldehyde + 45g grape sugar + 4g tartaric acid + 95 mL alcohol + 105 mL water to deposit about 0.3 $\mu$m thick silver layer onto the surface of tube portion of the main body of catheter. Following this, heat treatment was made again to cure to a desired hardness.

Besides, in this example, one with silver layer formed on a portion of catheter to be inserted into the human body was shown, but it goes without saying that the silver film may be formed on overall catheter.

With all of the catheters produced in the examples above, the metallic layer is uniformly adhered film-like on the surface of resin, yet the flexibility inherent in catheter is also not injured, and, even when inserted the catheter into the human body, it neither gives a stimulus to the human body, nor damages the inside of body cavity. Moreover, due to the sterile action or bactericidal action of metal ions, it also does not generate the infectious diseases, even if inserted into the human body over a long term.

As described above in detail, in accordance with the invention, excellent effects can be exerted that a metallic film, which does not give a stimulus to the human body when inserted into said human body and yet exhibits the bactericidal action or sterile action, can be formed on at least a portion of the main body of catheter to be inserted into the human body making it possible to prevent the infectious diseases through the bactericidal action or sterile action of such metals, that the metallic film formed on the main body of catheter is very thin, thus eliminating the hindrance an inserting into patient and alleviating the labors for the prevention from infectious diseases, for example, washing of bladder etc., and yet that the catheter can be provided in lower cost than the catheter adhered with metals having bactericidal action or sterile action onto conventional one with Teflon coat as mentioned above.

A method of producing catheter is provided, comprising the steps of molding a catheter with rubber or plastic, subjecting at least a portion of said catheter touching on human body to activation treatment, and then forming a metallic layer having bactericidal or sterile action on the main body of catheter by electroless plating treatment. Other methods and a catheter thus obtained are also claimed.

**Claims**

1. A method of producing catheter comprising the steps of molding a catheter with rubber or plastic, subjecting at least a portion of said catheter touching with human body to activation treatment, and then forming a metallic layer having bactericidal or sterile action on the main body of catheter by electroless plating treatment.

2. A method of producing catheter comprising the steps of molding a catheter with rubber or plastic, subjecting at least a portion of said catheter touching on human body to surface treatment with alkaline aqueous solution of alcohol followed by activation treatment, and then forming a metallic layer having bactericidal action or sterile action on the main body of catheter by electroless plating treatment.

3. The method of producing catheter according to Claim (1) or (2), wherein the catheter with the metallic layer formed by electroless plating treatment is treated at 30 to 159 °C under heat.

4. A method of producing catheter comprising the steps of molding a catheter with rubber or plastic, subjecting the surface layer of at least a portion of said catheter touching with human body to curing reaction to a state of half-cure, forming a metallic layer having bactericidal or sterile actions on said half-cured surface layer by electroless plating treatment and then curing said half-cured portion to a desired hardness.

5. A method of producing catheter comprising the steps of molding a catheter with rubber or plastic, subjecting the surface layer of at least a portion of said catheter touching with human body to curing reaction to a state of half-cure, forming a silver film on said half-cured surface layer by treatment of silver mirror reaction, and then curing said half-cured portion to a desired hardness.

6. The method of producing catheter according to any of Claim (1) through (5), wherein the surface of catheter molded with rubber or plastic is coated beforehand with Teflon.

7. A catheter obtained by any method of Claim (1) through (6).

# Fig. 1

# Fig. 2

# Fig. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 054 139 (CROSSLEY)<br>* the whole document * | 1-4 | A 61 M 25/00 |
| Y | PATENT ABSTRACTS OF JAPAN, vol. 11, no. 18 (C-398)[2465], 17 January 1987; & JP-A-61 194 183 (HITACHI) 28-08-1986<br>* abstract * | 1-4 | |
| A | DE-A-3 302 567 (STEIDLE)<br>* claims * | 1-6 | |
| A | PATENT ABSTRACTS OF JAPAN, vol. 6, no. 135 (C-115)[1013] 22 July 1982; JP-A-57 061 026 (TOSHIBA CORP.) 13-04-1982<br>* abstract * | 5 | |
| A | US-A-4 080 706 (HEILMAN et al.)<br>* abstract * | 6 | |

TECHNICAL FIELDS SEARCHED (Int. Cl.5)

A 61 M

The present search report has been drawn up for all claims

| Place of search | Date of completion of the search | Examiner |
|---|---|---|
| THE HAGUE | 03-03-1992 | MIR Y GUILLEN V. |